# FASCICULE DE BREVET EUROPEEN

(11) **EP 2 618 851 B1**
(45) Date de publication et mention de la délivrance du brevet: **16.07.2014**
(21) Numéro de dépôt: 11773743.7
(22) Date de dépôt: 23.09.2011
(51) Int. Cl.: A61L 2/14

(54) **DISPOSITIF DE STERILISATION PAR PLASMA FROID D'UN OBJET, TEL QU'UN DISPOSITIF MEDICAL, NOTAMMENT UN IMPLANT, ET PROCEDE METTANT EN OEUVRE CE DISPOSITIF**
VORRICHTUNG FÜR KALTE PLASMASTERILISATION EINES GEGENSTANDES WIE ETWA EINES MEDIZINPRODUKTS, IM BESONDEREN EINES IMPLANTATS, UND VERFAHREN ZUR VERWENDUNG DER VORRICHTUNG
DEVICE FOR COLD PLASMA STERILIZATION OF AN OBJECT, SUCH AS A MEDICAL DEVICE, PARTICULARLY AN IMPLANT, AND METHOD USING THIS DEVICE

(30) Priorité: 24.09.2010 FR 1057716
(43) Date de publication de la demande: 31.07.2013
(73) Titulaire: C.R.I.T.T. Materiaux, Depots Et Traitement De Surface, 08000 Charleville-Mézières (FR); Université De Reims Champagne-Ardenne, 51100 Reims (FR)
(72) Inventeur: POPOT, Jean-Marc, F-08000 Villers-Semeuse (FR); GELLE, Marie-Paule, F-51100 Reims (FR)
(74) Mandataire: Rhein, Alain
(86) Numéro de dépôt international: PCT/FR2011/052199
(87) Numéro de publication internationale: WO 2012/038669

(56) Documents cités:
- EP-A1- 1 477 188
- FR-A1- 2 850 280
- FR-A1- 2 888 118
- US-A- 5 453 125

## Description

L'invention concerne un dispositif de stérilisation, par plasma froid d'au moins un objet, tel qu'un dispositif médical, un implant ou autre, placé dans un contenant renfermant au moins un gaz ou un mélange gazeux approprié pour la génération d'un plasma froid. L'invention concerne encore un procédé de stérilisation mettant en oeuvre un tel dispositif de stérilisation.

La présente invention entre dans le domaine de la stérilisation d'objets, notamment des dispositifs médicaux, tels que des instruments chirurgicaux, des implants, voire des vêtements de protection utilisés dans ce domaine médical.

De manière connue, les plasmas froids sont induits en soumettant un gaz, ou un mélange gazeux, à un champ électrique, généré classiquement à moyenne ou haute fréquence, en opérant sous pression atmosphérique ou sous des pressions réduites de l'ordre de 10⁻¹ Millibars, soit 10 Pascals (Pa). Ils ont la particularité, suivant le gaz ou le mélange gazeux utilisé, de générer un rayonnement UV et des espèces actives, telles que de l'oxygène atomique, entre lesquels une synergie s'opère pour permettre la destruction de souches bactériennes.

Cette technologie constitue une alternative intéressante aux procédés de stérilisation classiques comme, par exemple, par chaleur humide comme l'autoclave, l'utilisation d'oxyde d'éthylène ou de rayons ionisants de type gamma, lorsque ces derniers ne se révèlent pas satisfaisants, que ce soit du point de vue de leur efficacité ou de leurs éventuels effets nocifs sur les objets ou dispositifs médicaux traités.

En effet, la stérilisation par autoclave ou chaleur humide, bien que définissant une méthode rapide, efficace et peu coûteuse, reste inappropriée au traitement d'objets en polymère, tandis que l'oxyde d'éthylène diffuse à l'intérieur des polymères et nécessite de laisser la surface traitée se désorber pendant au moins 24 heures.

Quant aux rayonnements ionisants gamma, bien qu'étant très efficace sur les matériaux polymères, ils présentent néanmoins l'inconvénient majeur d'un risque de dégradation des chaînes, entrainant une modification des caractéristiques du matériau, en raison de l'énergie de rayonnement élevée qu'ils diffusent.

Ainsi, les plasmas froids, dont la mise en oeuvre peut s'effectuer à température inférieure à 80°C, voire à température ambiante, conviennent tout particulièrement pour stériliser des objets ou des matières thermosensibles, qui risqueraient de se détériorer sous l'effet des hautes températures qu'impliquent certains procédés traditionnels; les plasmas froids permettent, par ailleurs, d'éviter un certain nombre d'autres agressions physicochimiques pouvant parfois être constatées avec ces derniers, notamment à l'égard d'objets en matériaux polymères.

Enfin, la stérilisation par plasmas froids présente l'avantage de ne générer que des effluents gazeux aussi bien inoffensifs pour l'opérateur, que totalement respectueux de l'environnement.

Plusieurs documents de l'art antérieur décrivent des procédés ou des dispositifs de stérilisation qui reposent sur la technologie des plasmas froids.

Le document WO 00/72889 présente un système et un procédé de stérilisation par plasma à basse température, dans lequel il est fait appel à un gaz ne nécessitant pas de présenter une action stérilisante intrinsèque, celle-ci résultant du passage d'un flux dudit gaz à travers un champ électrique généré par micro-ondes, ledit gaz comprenant de l'oxygène sous forme moléculaire et une espèce atomique ou moléculaire capable d'émettre un rayonnement ultra-violet après avoir été excitée.

La stérilisation s'opère à une température inférieure à 50°C, en plaçant les objets ou matières concernés à l'intérieur d'une enceinte de stérilisation reliée à une pompe à vide et à une source de plasma, et en les soumettant tels quels à un flux dudit plasma.

Le document WO 00/54819 concerne un procédé et un dispositif de stérilisation par plasma dans lequel les objets à traiter sont placés dans une enceinte de stérilisation sensiblement sous pression atmosphérique, à l'intérieur de laquelle on introduit un ou plusieurs mélanges gazeux non biocides dont l'un au moins contient de l'humidité.

On crée ensuite un plasma produisant des espèces actives, à partir d'un des mélanges gazeux, en générant, au moyen d'une alimentation haute tension, une décharge électrique entre deux électrodes placées dans ladite enceinte de stérilisation, et on achemine ledit plasma et ladite humidité directement vers la surface des objets à traiter.

Le mélange gazeux préconisé par ce document contient au moins 10% d'oxygène et 10% d'azote et est de préférence constitué par de l'air ambiant.

En fait, bien que ces documents décrivent des procédés de stérilisation par plasmas froids et/ou des dispositifs pour leur mise en oeuvre, aptes à traiter efficacement des objets en les soumettant directement au flux de plasma, aucun d'entre eux n'aborde et ne permet de résoudre le problème du conditionnement de ces objets, étape au cours de laquelle une nouvelle contamination par des souches bactériennes est souvent constatée.

Les procédés précités ne peuvent donc être considérés comme totalement satisfaisants puisqu'ils se limitent à stériliser les objets à proprement parler, sans prévoir de solution permettant d'assurer la préservation du caractère stérile lors et après conditionnement.

Une solution qui a été proposée pour résoudre ce problème, et décrite notamment dans le document US 4 321 232, consiste à réaliser une stérilisation sur un objet déjà conditionné.

L'emballage utilisé présente un caractère poreux autorisant la pénétration du plasma, généré par le biais de moyens classiques, à l'extérieur de cet emballage.

On a cependant pu constater, que cette solution n'apporte pas non plus entière satisfaction, car elle nécessite un temps de traitement de l'objet bien supérieur, pour atteindre un degré de stérilisation comparable à celui obtenu avec un flux de plasma directement appliqué.

En effet, l'épaisseur de la membrane de l'emballage poreux empêche la diffusion des rayons ultra-violets jusqu'aux objets, et provoque par ailleurs des phénomènes de recombinaison des espèces actives, qui ne sont alors plus capables d'assurer leur rôle de manière optimale.

Il est encore connu par le document FR 2 850 280 du même déposant, un procédé de stérilisation par plasma froid de dispositifs médicaux, d'implants ou autres, dans lequel on vient placer dans une enceinte de stérilisation un sachet scellé rempli de gaz. Ce sachet est ensuite soumis à un champ électrique qui va induire un plasma à l'intérieur du sachet en agissant sur la pression du gaz emprisonné dans ledit sachet.

Enfin, il est encore connu pour la création d'un plasma, l'utilisation d'un générateur à résonance cyclotronique des électrons (ECR), qui par la génération de micro-ondes couplé d'une part, à la génération d'un champ magnétique et, d'autre part, à l'introduction d'un gaz approprié va générer un plasma froid.

Toutes ces solutions antérieurement connues n'apportent pas pleinement satisfaction quant au résultat obtenu par rapport à celui attendu et les moyens mis en oeuvre.

Par conséquent, l'objet de la présente invention est de proposer une nouvelle solution pour pallier, non seulement le problème de la préservation du caractère stérile d'objets lors de leur conditionnement, mais, en outre, d'obtenir un résultat répétitif en terme de qualité de stérilisation grâce à des moyens mis en oeuvre optimisés.

Aussi, c'est dans le cadre d'une démarche inventive qu'il a été imaginé la combinaison de moyens de polarisation d'un support avec un générateur de champ magnétique, pour assurer, à la fois :
- une agitation moléculaire suffisante du gaz contenu dans un contenant placé sur le support polarisé à l'intérieur d'une enceinte de stérilisation soumise à un vide secondaire ; et
- une concentration optimale sur ce contenant d'un flux de micro-ondes émanant d'un générateur à résonance cyclotronique d'électrons.

Cela permet, finalement, d'allumer le plasma uniquement dans le contenant et non dans l'enceinte.

Ainsi, l'invention concerne un dispositif de stérilisation par plasma froid pour la stérilisation d'au moins un objet, tel qu'un dispositif médical, notamment un implant, placé dans un contenant renfermant au moins un gaz ou un mélange gazeux approprié pour la génération d'un plasma froid, ledit dispositif comprenant :
- une enceinte fermée dans laquelle est ménagé un support pour la réception dudit contenant ;
- des moyens (5) pour soumettre l'enceinte à un vide secondaire de l'ordre de 10⁻³ à 10⁻⁷ millibars ;
   caractérisé en ce qu'il comporte encore :
- un générateur à résonance cyclotronique des électrons pour générer dans l'enceinte un flux de micro-ondes couplé à un champ magnétique au moins dans une phase d'initialisation du plasma froid ;
- des moyens de polarisation dudit support du contenant.

Selon une autre caractéristique de l'invention, ledit dispositif comporte encore au moins un moyen d'injection dans le contenant d'un gaz ou d'un mélange gazeux approprié pour la génération d'un plasma froid à une pression supérieure à la pression dans l'enceinte de stérilisation.

Selon l'invention le contenant comporte au moins un premier opercule d'injection d'un gaz ou d'un mélange gazeux approprié pour la génération d'un plasma froid.

Selon une autre caractéristique, ledit contenant comporte encore au moins un second opercule d'échappement de gaz.

Selon encore une autre caractéristique, le moyen d'injection de gaz et/ou l'opercule d'échappement du contenant constituent des moyens de contrôle d'une pression de gaz constante ou quasi constante dans ledit contenant.

En particulier, au travers de ce second opercule on peut gérer la pression dans ledit contenant pour éviter sa détérioration, tout en assurant le maintien du plasma froid.

L'invention concerne encore un procédé de stérilisation mettant en oeuvre un dispositif de stérilisation selon l'invention, caractérisé en ce que :
- on dispose au moins un contenant renfermant au moins un objet, notamment un dispositif médical, sur un support dans une enceinte de stérilisation ;
- on ferme hermétiquement ladite enceinte et on la soumet à un vide secondaire ;
- on soumet le support du contenant à une polarisation, notamment par radio fréquence ;
- au travers d'un générateur ECR et au moins dans une phase d'initialisation du plasma froid, on soumet le contenant à un flux de micro-ondes couplé à un champ magnétique ;
- par injection dans le contenant d'au moins un gaz ou mélange gazeux approprié pour la génération d'un plasma froid, on maintien dans le contenant une pression supérieure à la pression dans l'enceinte de stérilisation.

Selon une autre caractéristique du procédé selon l'invention, après allumage du plasma froid dans le contenant on maintien dans ce dernier une pression constante.

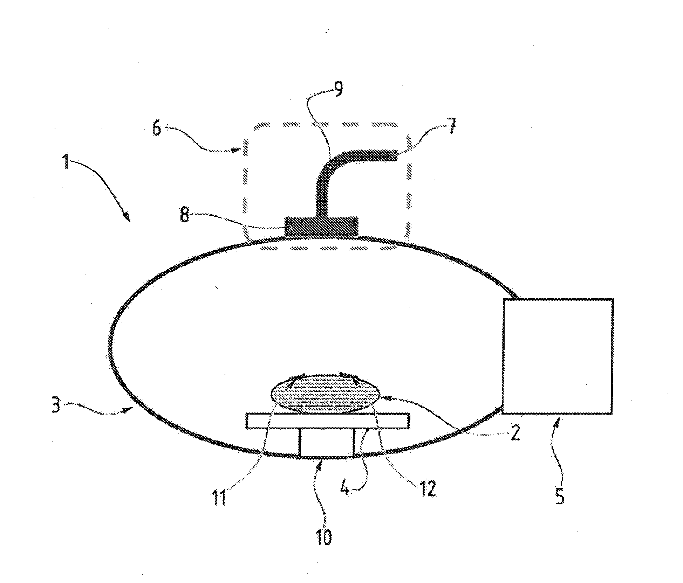

D'autres caractéristiques et avantages de l'invention ressortiront de la description détaillée qui va suivre des modes de réalisation non limitatifs de l'invention, en référence à la figure annexée représentant de façon schématisée une vue en coupe verticale d'un mode de réalisation d'un dispositif de stérilisation selon l'invention.

La présente invention entre dans le domaine de la stérilisation d'objets, notamment de dispositif médicaux, tels que des instruments chirurgicaux, des implants ou similaire. L'invention s'intéresse plus particulièrement à la stérilisation de ces objets par ailleurs contenus dans un contenant adapté, correspondant par exemple à leur emballage, et apte à renfermer au moins un gaz ou un mélange gazeux approprié pour la génération d'un plasma froid.

A ce propos, il convient de préciser que si ce contenant peut avant même l'opération de stérilisation contenir un tel gaz ou mélange gazeux, celui-ci peut encore être injecté au cours de cette stérilisation.

L'invention concerne plus particulièrement un dispositif de stérilisation 1 par plasma froid en vue de la stérilisation de tels objets disposés dans un contenant 2.

Ainsi, ce dispositif de stérilisation 1 comporte une enceinte de stérilisation 3 fermée avec, cependant, des moyens d'accès appropriés, tels une porte ou un sas, pour pouvoir y déposer les objets à stériliser. D'ailleurs, dans cette enceinte 3 est prévu un support 4 adapté, par exemple sous forme d'un plateau, pour recevoir le contenant 2 en question.

L'enceinte 3 est préférentiellement étanche de sorte que, durant le processus de génération de plasma, on puisse faire le vide secondaire à l'intérieur. Plus particulièrement, cette enceinte 3 doit être à même de supporter une pression de l'ordre de 10⁻³ à 10⁻⁷ millibars, soit de l'ordre de 10⁻¹ à 10⁻⁵ Pascals (Pa), notamment de 10⁻⁵ millibars, soit 10⁻³ Pascals (Pa), ces valeurs se situant dans la plage du vide, dit « vide secondaire ».

A ce propos, ledit dispositif 1 comporte encore des moyens 5 pour soumettre l'enceinte 3 à un tel vide secondaire. Par exemple, sur l'une des parois de cette enceinte 3 peut être reliée hermétiquement à un dispositif de pompage à vide. Ainsi avant de procéder à une stérilisation, on vient vider l'air contenu dans l'enceinte 3 suite à l'introduction du contenant 2 renfermant l'objet à stériliser.

Le dispositif 1 comporte également un générateur ECR 6 ou générateur à résonance cyclotronique des électrons comportant des moyens générateurs de micro-ondes 7 et des moyens générateurs d'un champ magnétique 8 pour soumettre le contenant 2 dans l'enceinte 3 à un flux de micro-ondes couplé à un champ magnétique, ceci au moins dans une phase d'initialisation du plasma froid.

Plus particulièrement, les moyens générateurs de micro-ondes 7 comportent un guide d'ondes 9 agencé pour pouvoir orienter et concentrer lesdites micro-ondes émises en direction de l'enceinte 3, et plus particulièrement vers le contenant 2. Selon un mode de réalisation de l'invention, ce générateur à micro-ondes 7 émet des micro-ondes d'une puissance de quelques dizaines à centaines de Watts et préférentiellement de l'ordre de 250W.

Les moyens générateurs d'un champ magnétique 8 de l'ECR 6 peuvent, quant à eux, être constitués par un solénoïde pourvu d'un bobinage de fils électriques ou des aimants permanents et par des aimants multipolaires de fonctionnement classiquement connus.

Tel que visible sur la figure, ces moyens générateurs d'un champ magnétique 8 viennent entourer le guide d'ondes 9 lequel conduit les micro-ondes dans l'enceinte 3 au-dessus du support 4.

Selon l'invention, le dispositif de stérilisation comporte, en combinaison des moyens exposés plus haut, des moyens de polarisation 10 du support 4 au contenant 2.

Plus particulièrement, au travers de cette polarisation il en résulte une agitation moléculaire dans le contenant 2, qui combinée à la focalisation du flux de micro-ondes généré par l'ECR 6, a pour avantage d'assurer l'allumage du plasma froid dans le contenant 2 et non dans l'enceinte, par ailleurs soumise à un vide secondaire.

Préférentiellement, les moyens de polarisation 10 sont du type par radio fréquence de manière à exciter le gaz ou mélange gazeux et, ainsi, assurer l'agitation moléculaire qui, au travers de la focalisation des micro-ondes par champ magnétique, conduit à ioniser le gaz et induire le plasma froid dans le contenant. A titre d'exemple, cette polarisation par radio fréquence peut avoir une puissance de quelques centaines de Watts, préférentiellement de l'ordre de 250 Watts, avec une tension de polarisation de l'ordre de 200 Volts.

Il convient de préciser que le gaz ou mélange gazeux que doit contenir le contenant 2 est de type plasmagène. Il peut être constitué par de l'air ou dans le cas d'un mélange gazeux, à base, par exemple, d'oxygène ou d'argon.

De plus, le contenant 2 peut contenir un tel gaz ou mélange gazeux plasmagène au moment de le placer dans l'enceinte de stérilisation 3 et/ou il peut y être injecté en cours d'opération de stérilisation. Comme cela sera expliqué plus en avant, au travers d'une telle injection en cours de processus de stérilisation il est possible de maintenir dans le contenant une pression de gaz suffisante pour maintenir le plasma froid qui, sans quoi, risque de s'éteindre trop tôt et conduire à une stérilisation incomplète.

On remarquera que le contenant 2 peut être constitué par un sachet en matériau polymère non poreux au gaz ou mélange de gaz qu'il est amené à contenir. Un tel sachet peut encore être conçu conformément à des normes spécifiques en fonction de son contenu. Il en est notamment ainsi, en ce qui concerne les dispositifs médicaux, implants ou autres, domaines auxquels s'intéresse plus particulièrement la présente invention.

Selon une autre caractéristique de l'invention, ledit dispositif de stérilisation 1 peut comporter, encore, au moins un moyen d'injection d'un gaz ou mélange gazeux approprié pour la génération d'un plasma froid dans le contenant 2 à une pression supérieure à la pression dans l'enceinte de stérilisation 3. D'ailleurs, le contenant 2 comporte préférentiellement au moins un premier opercule 11 pour permettre cette injection de gaz. Ce moyen d'injection de gaz peut être constitué par une buse d'injection raccordée à un système de distribution de gaz adapté.

De plus, ledit contenant 2 peut être pourvu d'au moins un second opercule 12 d'échappement de gaz pour éviter sa détérioration en cas de surpression, tout assurant la pression nécessaire au maintien du plasma froid dans ce contenant.

Avantageusement, le moyen d'injection de gaz et/ou l'opercule d'échappement 12 du contenant 2 constituent des moyens de contrôle d'une pression de gaz constante ou quasi constante dans ledit contenant.

Le gaz est préférentiellement injecté dans ledit contenant 2 sous une pression inférieure à 100 Pa, tenant compte du fait que l'enceinte de stérilisation 3 est à une pression inférieure à 10⁻³ Pa. Cette différence de pression entre l'enceinte de stérilisation 3 et l'intérieur du contenant 2 va permettre l'induction du plasma à l'intérieur du contenant 2 tout en évitant un éventuel amorçage d'un plasma dans l'enceinte 3.

A noter que le gaz ou le mélange gazeux peut être injecté de manière continue ou discontinue à l'intérieur du contenant 2 en fonction de l'évolution des flux de gaz dans le contenant 2 pendant la réaction ou encore dans un but d'entretien du plasma à l'intérieur dudit contenant 2.

Selon un mode de réalisation préféré de l'invention, le premier opercule 11 d'entrée de gaz, tout comme l'opercule d'échappement 12, est poreux et conçu pour agir telle une valve, en autorisant, pour le premier 11, l'injection de gaz et pour l'opercule d'échappement 12, comme l'indique son nom, la sortie de gaz.

Avantageusement, ce second opercule 12 sert encore de soupape qui va permettre de réguler la pression à l'intérieur dudit contenant durant la procédure de stérilisation en expulsant un trop plein de gaz, afin d'empêcher la détérioration de ce contenant, tout en évitant l'extinction du plasma.

Il est important de noter que le débit du gaz ou du mélange gazeux injecté dans le contenant 2 dépend de nombreux paramètres tels que :
- la pression dans l'enceinte 3;
- la pression dans le contenant 2;
- le volume du contenant 2 ;
- etc...

Selon un mode de réalisation de l'invention, le dispositif 1 comporte des moyens d'évaluation et de régulation (non représentés sur le dessin) de la pression régnant à l'intérieur du contenant 2. Selon un exemple de réalisation, les moyens pour évaluer la pression peuvent être constitués par un ou plusieurs capteurs optiques aptes à suivre l'évolution du volume du contenant, en l'occurrence lorsqu'il se présente sous forme d'un sachet, pour déterminer et/ou estimer la pression en son sein. Bien évidemment, on peut encore envisager de faire appel à d'autres capteurs de pression disposés sur ou dans le contenant, éventuellement couplés à des moyens de transmission sans fils adaptés, pour connaitre cette pression dans le contenant et gérer l'injection de gaz. De même on peut encore songer équiper la buse d'injection de gaz, voire une canule raccordée à l'opercule d'échappement 12 du contenant 2 de tels moyens d'évaluation de pression.

Quant aux moyens de régulation, ils peuvent se présenter sous forme d'une unité de gestion capable d'intervenir, par exemple, sur le moyen d'injection de gaz dans le contenant en fonction de l'information qui est délivrée à cette unité de gestion par les dits moyens d'évaluation précités.

L'invention concerne également un procédé de stérilisation mettant en oeuvre un tel dispositif de stérilisation 1, consistant en ce que :
- on dispose au moins un contenant 2 renfermant au moins un objet, notamment un dispositif médical, sur un support 4 dans une enceinte de stérilisation 3;
- on ferme hermétiquement ladite enceinte 3 et on la soumet à un vide secondaire ;
- on soumet le support 4 au contenant 2 à une polarisation, notamment par radio fréquence ;
- au travers d'un générateur ECR 6 et au moins dans une phase d'initialisation du plasma froid, on soumet le contenant 2 à un flux de micro-ondes couplé à un champ magnétique ;
- par injection dans le contenant 2 d'un moins un gaz ou mélange gazeux approprié pour la génération d'un plasma froid, on maintien dans le contenant 2 une pression supérieure à la pression dans l'enceinte de stérilisation 3.

Avantageusement, après allumage du plasma froid dans le contenant 2, on maintient dans ce dernier une pression constante, à savoir stable à une valeur donnée, ou bien sensiblement constante, à savoir variant de plus ou moins 1 à 10% autour de ladite valeur donnée.

C'est donc l'effet combiné d'un générateur de micro-ondes 7 couplé à un champ magnétique induit par les moyens de génération 8, ainsi que la polarisation par radio fréquence du support 4 sur lequel repose ledit contenant 2 qui conduit à « allumer » le plasma uniquement dans le contenant 2 et non dans l'enceinte de stérilisation 3 qui est soumise à un vide secondaire.

## Revendications

1. Dispositif de stérilisation (1) par plasma froid pour la stérilisation d'au moins un objet, tel qu'un dispositif médical, notamment un implant, placé dans un contenant (2) renfermant au moins un gaz ou un mélange gazeux approprié pour la génération d'un plasma froid, ledit dispositif comprenant :
- une enceinte (3) fermée dans laquelle est ménagé un support (4) pour la réception dudit contenant (2) ;
- des moyens (5) pour soumettre l'enceinte à un vide secondaire de l'ordre de 10⁻³ à 10⁻⁷ millibars (ie. 10⁻¹ à 10⁻⁵ Pascals) ;
**caractérisé en ce qu'**il comporte encore :
- un générateur à résonance cyclotronique des électrons (6) pour générer dans l'enceinte un flux de micro-ondes couplé à un champ magnétique au moins dans une phase d'initialisation du plasma froid ;
- des moyens de polarisation (10) dudit support (4) du contenant (2).

2. Dispositif (1) selon la revendication 1, **caractérisé par le fait que** les moyens de polarisation (10) sont du type par radio fréquence.

3. Dispositif (1) selon la revendication 1 ou 2, **caractérisé par le fait qu'**il comporte encore au moins un moyen d'injection dans le contenant (2) d'un gaz ou d'un mélange gazeux approprié pour la génération d'un plasma froid à une pression supérieure à la pression dans l'enceinte de stérilisation (3).

4. Dispositif (1) selon l'une quelconque des revendications précédentes, **caractérisé par le fait que** le contenant (2) comporte au moins un premier opercule (11) d'injection d'un gaz ou d'un mélange gazeux approprié pour la génération d'un plasma froid.

5. Dispositif (1) selon la revendication 4, **caractérisé par le fait que** ledit contenant (2) comporte au moins un second opercule (12) d'échappement de gaz.

6. Dispositif (1) selon la revendication 3 ou 5, **caractérisé par le fait que** le moyen d'injection de gaz et/ou l'opercule d'échappement (12) du contenant (2) constituent des moyens de contrôle d'une pression de gaz constante ou quasi constante dans ledit contenant (2).

7. Dispositif (1) selon l'une quelconque des revendications précédentes, **caractérisé par le fait qu'**il comporte des moyens d'évaluation et de régulation de la pression régnant à l'intérieur du contenant (2).

8. Procédé de stérilisation mettant en oeuvre un dispositif de stérilisation (1) selon l'une quelconque des revendications 1 à 7, **caractérisé en ce que** :
- on dispose au moins un contenant (2) renfermant au moins un objet, notamment un dispositif médical, sur un support (4) dans une enceinte de stérilisation (3);
- on ferme hermétiquement ladite enceinte (3) et on la soumet à un vide secondaire ;
- on soumet le support (4) du contenant (2) à une polarisation, notamment par radio fréquence ;
- au travers d'un générateur à résonance cyclotronique des électrons (6) et au moins dans une phase d'initialisation du plasma froid, on soumet le contenant (2) à un flux de micro-ondes couplé à un champ magnétique ;
- par injection dans le contenant (2) d'un moins un gaz ou mélange gazeux approprié pour la génération d'un plasma froid on maintien dans le contenant (2) une pression supérieure à la pression dans l'enceinte de stérilisation.

9. Procédé de stérilisation selon la revendication 8, **caractérisé par le fait qu'**après allumage du plasma froid dans le contenant (2), on maintient dans ce dernier une pression constante.

## Patentansprüche

1. Kaltplasma-Sterilisationsvorrichtung (1) für die Sterilisation von zumindest einem Objekt, wie beispielsweise einer medizinischen Vorrichtung, nämlich eines Implantats, der in einem Behälter (2) aufgestellt ist, der mindestens ein zum Erzeugen eines kalten Plasmas geeignetes Gas oder Gasgemisch enthält, wobei die Vorrichtung Folgendes umfasst:
- eine geschlossene Kammer (3), in der eine Halterung (4) zur Aufnahme des besagten Behälters (2) angeordnet ist;
- Mittel (5), um die Kammer einem sekundären Vakuum von etwa 10⁻³ bis 10⁻⁷ mbar (d.h. 10⁻¹ bis 10⁻⁵ Pa) zu unterziehen;
**dadurch gekennzeichnet, dass** sie ferner Folgendes umfasst:
- einen Elektronen-Zyklotron-Resonanz-Generator (6), um in der Kammer einen Mikrowellenstrom zu erzeugen, der zumindest in einer Initialisierungsphase des kalten Plasmas mit einem Magnetfeld gekoppelt ist;
- Mittel zum Polarisieren (10) der Halterung (4) des Behälters (2).

2. Vorrichtung (1) nach Anspruch 1, **dadurch gekennzeichnet, dass** die Mittel zum Polarisieren (10) der Art mit Radiofrequenz sind.

3. Vorrichtung (1) nach Anspruch 1 oder 2, **dadurch gekennzeichnet, dass** sie ferner zumindest eine Einrichtung umfasst, um in den Behälter (2) ein Gas oder ein Gasgemisch einzuspritzen, das geeignet ist, um ein kaltes Plasma bei einem Druck, der höher als der Druck in der Sterilisationskammer (3) ist, zu erzeugen.

4. Vorrichtung (1) nach einem der vorhergehenden Ansprüche, **dadurch gekennzeichnet, dass** der Behälter (2) zumindest eine erste Schließklappe (11) zum Einspritzen eines zum Erzeugen eines kalten Plasmas geeignetes Gases oder Gasgemisches umfasst.

5. Vorrichtung (1) nach Anspruch 4, **dadurch gekennzeichnet, dass** der Behälter (2) mindestens eine zweite Schließklappe (12) zum Ablassen von Gasen umfasst.

6. Vorrichtung (1) nach Anspruch 3 oder 5, **dadurch gekennzeichnet, dass** die Gaseinspritzeinrichtung und/oder das Ablass-Schließklappe (12) des Behälters (2) Mittel zum Steuern eines konstanten oder nahezu konstanten Gasdrucks in dem besagten Behälter (2) bilden.

7. Vorrichtung (1) nach einem der vorhergehenden Ansprüche, **dadurch gekennzeichnet, dass** sie Mittel zum Auswerten und Regeln des innerhalb des Behälters (2) herrschenden Drucks umfasst.

8. Sterilisationsverfahren zum Umsetzen einer Sterilisationsvorrichtung (1) nach einem der Ansprüche 1 bis 7, **dadurch gekennzeichnet, dass**:
- mindestens ein Behälter (2), der mindestens einen Gegenstand, nämlich eine medizinische Vorrichtung, enthält, auf eine Halterung (4) in einer Sterilisationskammer (3) angeordnet wird;
- die besagte Kammer (3) hermetisch geschlossen und einem sekundären Vakuum ausgesetzt wird;
die Halterung (4) des Behälters (2) einer Polarisation, nämlich durch Radiofrequenz unterzogen wird;
- der Behälter (2) über einen Elektronen-Zyklotron-Resonanz-Generator (6) zumindest in einer Initialisierungsphase des kalten Plasmas einem mit einem Magnetfeld gekoppelten Mikrowellenstrom unterzogen wird;
- durch Injektion in den Behälter (2) mindestens eines zum Erzeugen eines kalten Plasmas geeigneten Gases oder Gasgemisches wird in dem Behälter (2) ein Druck aufrechterhalten, der höher ist als der Druck in der Sterilisationskammer.

9. Sterilisationsverfahren nach Anspruch 8, **dadurch gekennzeichnet, dass** nach dem Zünden des kalten Plasmas in dem Behälter (2) ein konstanter Druck in diesem Letzteren aufrechterhalten wird.

## Claims

1. Cold-plasma sterilization device (1) for the sterilization of at least one object, such as a medical device, namely an implant, placed in a container (2) containing at least one gas or a gas mixture suitable for generating a cold plasma, said device comprising:
- a closed chamber (3), in which a support (4) is provided for receiving said container (2);
- means (5) for subjecting the chamber to a secondary vacuum in the range of 10⁻³ to 10⁻⁷ mbar (i.e. 10⁻¹ to 10⁻⁵ Pascal);
wherein it also comprises:
- an electron cyclotron resonance generator (6) for generating in the chamber a micro-wave flow coupled to a magnetic field at least in a cold-plasma initializing phase;
- means for polarizing (10) said support (4) of the container (2).

2. Device (1) according to claim 1, wherein the polarization means (10) are of the radiofrequency type.

3. Device (1) according to claim 1 or 2, wherein it also comprises at least one means for injecting into the container (2) a gas or a gas mixture suitable for generating a cold plasma at a pressure higher than the pressure in the sterilization chamber (3).

4. Device (1) according to any of the preceding claims, wherein the container (2) comprises at least one first cover (11) for injecting a gas or a gas mixture suitable for generating a cold plasma.

5. Device (1) according to claim 4, wherein said container (2) comprises at least one second cover (12) for the exhaust of gases.

6. Device (1) according to claim 3 or 5, wherein the gas injection means and/or the exhaust cover (12) of the container (2) constitute means for controlling a constant or almost constant gas pressure in said container (2).

7. Device (1) according to any of the preceding claims, wherein it includes means for evaluating and regulating the pressure reigning inside said container (2).

8. Sterilization method implementing a sterilization device (1) according to any of claims 1 to 7, wherein:
- at least one container (2) containing at least one object, namely a medical device, is arranged on a support (4) in a sterilization chamber (3);
- said chamber is tightly sealed (3) and it is subjected to a secondary vacuum;
- the support (4) of the container (2) is subjected to a polarization, namely by radiofrequency;
- through an electron cyclotron resonance generator (6) and at least in an initialization stage of the cold plasma, the container (2) is subjected to a microwave flow coupled to a magnetic field;
- by injecting into the container (2) a least one gas or gas mixture suitable for generating a cold plasma, a pressure higher than the pressure in the sterilization chamber is maintained in the container (2).

9. Sterilization method according to claim 8, wherein after ignition of the cold plasma in the container (2), a constant pressure is maintained in the latter.
